# FASCICULE DE BREVET EUROPEEN

(11) **EP 2 085 041 B1**
(45) Date de publication et mention de la délivrance du brevet: **06.10.2010**
(21) Numéro de dépôt: 08101066.2
(22) Date de dépôt: 29.01.2008
(51) Int. Cl.: A61B 17/88, B25B 23/142, A61B 19/00, A61C 1/18, A61C 8/00

(54) **Outil dynamometrique a usage medical**
Kraftmesswerkzeug für den medizinischen Einsatz
Dynamometric tool for medical use

(43) Date de publication de la demande: 05.08.2009
(73) Titulaire: Hader SA, 2300 La Chaux-de-Fonds (CH)
(72) Inventeur: Miletto, Philippe, 1317, Orny (CH); Gross, Silver, 2300, La Chaux-de-Fonds (CH)
(74) Mandataire: GLN

(56) Documents cités:
- WO-A-2007/137961
- US-A- 5 224 403
- US-A- 5 816 809

## Description

### Domaine technique

La présente invention se rapporte au domaine des outils à usage médical. Elle concerne, plus particulièrement, un outil dynamométrique destiné à serrer et à desserrer des vis ou divers objets comportant un pas de vis, typiquement au cours d'une intervention chirurgicale.

Il est, en effet, particulièrement important d'éviter d'appliquer des couples de serrage non contrôlés, par exemple dans le cas où une plaque est fixée sur un os pour réparer une fracture. Si le serrage appliqué est excessif, cela peut conduire à écraser l'os et à l'endommager davantage.

### Etat de la technique

On connaît, dans l'état de la technique, des outils dynamométriques pour le vissage, et parfois le dévissage, de différents objets, notamment de vis pour la fixation d'éléments de reconstruction dans la chirurgie réparatrice. A titre d'exemple d'application, des plaques peuvent être vissées dans des os fracturés en vue de faciliter leur réparation. Un tel outil comprend:
- un corps de préhension pour que le chirurgien puisse le manipuler, et
- un porte-instrument pour être rendu solidaire en rotation d'un instrument conformé pour coopérer avec l'objet à visser.

Il existe deux principaux types d'outils dynamométriques. Un premier fournit une limitation du couple de serrage, par la mise en place d'une butée de sécurité qui rend solidaire en rotation le corps de préhension et le porte-instrument tant que le couple maximal choisi n'est pas atteint et qui se rond lorsqu'une charge de rupture déterminée et correspondant au couple maximal, est atteinte. Un tel type d'outil est par exemple décrit dans le document US 5,368,480. Un organe de limitation de couple est inséré entre le corps de préhension et le porte-instrument. Cet organe comporte une pluralité d'ergots dimensionnés de manière à former la butée mentionnée ci-dessus et à s'écraser lorsque le couple de serrage voulu est atteint.

Toutefois, ces ergots subissent une déformation plastique, c'est-à-dire irréversible, ce qui a pour conséquence que chacun des ergots ne peut être utilisé qu'une seule fois. Bien sûr, le fait que l'organe de limitation de couple comporte une pluralité d'ergots permet d'effectuer plusieurs serrages à couple maximal, mais seulement un nombre très limité. De plus, il est difficile de contrôler avec précision le couple maximal de serrage, car le seuil de déformation plastique dépend d'un grand nombre de paramètres et peut être influencé par de légères différences intervenues lors de la fabrication de l'organe de limitation de couple.

Le second type d'outils dynamométriques permet d'effectuer un grand nombre de serrage au couple maximum, sans avoir à intervenir sur l'outil, grâce à une sorte de système d'encliquetage. Dans certains outils de ce type, le porte-instrument se prolonge par un arbre pivotant dans le corps de préhension, l'arbre étant relié à friction au corps de préhension. La friction est assurée par deux dentures Breguet, c'est-à-dire des dentures de chant en forme de scie, l'une étant solidaire du porte-instrument et l'autre étant solidaire en rotation du corps de préhension. Des ressorts pressent les deux dentures Breguet l'une contre l'autre de manière à rendre solidaires en rotation le porte-instrument et le corps de préhension. Quand le couple de serrage est supérieur aux frictions imposées entre les deux dentures Breguet par les ressorts, celles-ci frottent l'une contre l'autre et s'échappent. Le porte-instrument n'est alors plus entraîné en rotation par le corps de préhension. Le couple de serrage maximal applicable peut être réglé en modulant la pression exercée par les ressorts.

Les frictions créées entre les dentures sont particulièrement importantes et il est nécessaire, pour obtenir une précision et une longévité acceptables de l'outil, que les dentures Breguet soient métalliques. Elles, ainsi que les ressorts, sont réalisées en un acier inoxydable, permettant une utilisation chirurgicale aussi hygiénique que possible. Il est toutefois nécessaire de graisser les parties métalliques en friction, ce qui n'est pas très satisfaisant d'un point de vue sanitaire, puisqu'un risque de coulée de graisse à l'extérieur de l'outil existe lors des opérations de stérilisation. En outre, la précision d'un tel outil est peu satisfaisante (+/- 10%) et il est nécessaire d'effectuer régulièrement des calibrations.

De plus, lorsque le couple de serrage maximal est atteint et que les dentures s'échappent l'une de l'autre, cela provoque des sauts dans la direction longitudinale de l'outil, ce qui n'est pas agréable pour le chirurgien et qui peut lui faire faire un geste maladroit. Par ailleurs, les matériaux utilisés pour la réalisation de cet outil le rendent lourd et peu pratique.

La présente invention concerne les outils dynamométriques du second type, permettant d'effectuer un grand nombre de serrage au couple maximum, sans intervention sur l'outil. Le but recherché est de proposer un outil dynamométrique exempt des inconvénients susmentionnés et qui, en particulier, est précis, léger, facile à manipuler. Un tel outil reprenant les caractéristiques du préambule de la revendication 1, est notamment connu du document W02007/137961. La stabilité du couple de serrage maximal est un élément important dans la qualité d'un tel outil, particulièrement par rapport aux traitements de stérilisation qui imposent parfois des conditions sévères. L, l'invention a également pour but de proposer un outil amélioré dont le couple de serrage maximal n'est pas affecté par les traitements subis lors des étapes de stérilisation.

### Divulgation de l'invention

De façon plus précise, l'invention concerne un outil dynamométrique à usage médical, d'axe longitudinal AA comportant:
- un corps de préhension creux comprenant une paroi intérieure présentant une succession de creux orientés selon l'axe AA et dont la section est ovoïde,
- un porte-instrument pour être rendu solidaire en rotation d'un instrument conformé pour coopérer avec un objet à visser, ledit porte-instrument se prolongeant par un arbre d'axe AA pivotant à l'intérieur du corps de préhension.

Le porte-instrument est relié à friction au corps de préhension au moyen d'une pluralité d'organes élastiques disposés entre l'arbre et le corps de préhension. Chaque organe élastique comporte une pluralité de lamelles disposées principalement selon des directions non radiales et déformables élastiquement selon une direction essentiellement radiale, les extrémités des lamelles formant des patins destinés à coopérer avec les creux de la paroi intérieure.

Selon l'invention, afin d'éviter que la dilatation causée par le chauffage de la stérilisation entraine une modification des propriétés élastiques des organes élastiques et un dérèglement du couple de serrage maximal, les organes élastiques sont montés fous sur l'arbre et présentent un jeu entre l'arbre et les organes élastiques, d'une part, et entre les organes élastiques et la paroi intérieure, d'autre part.

### Brève description des dessins

D'autres détails apparaîtront plus clairement à la lecture de la description qui suit, faite en regard des dessins annexés, dans lesquels :
- la figure 1 est une vue en coupe transversale du corps de préhension et d'un organe élastique particulièrement adapté pour la mise en oeuvre de l'invention, la figure comportant un plan rapproché montrant en détail l'interaction entre le corps de préhension et un organe élastique, et
- la figure 2 est une vue en coupe longitudinale du dispositif selon l'invention d'une zone de cette partie.

### Mode(s) de réalisation de l'invention

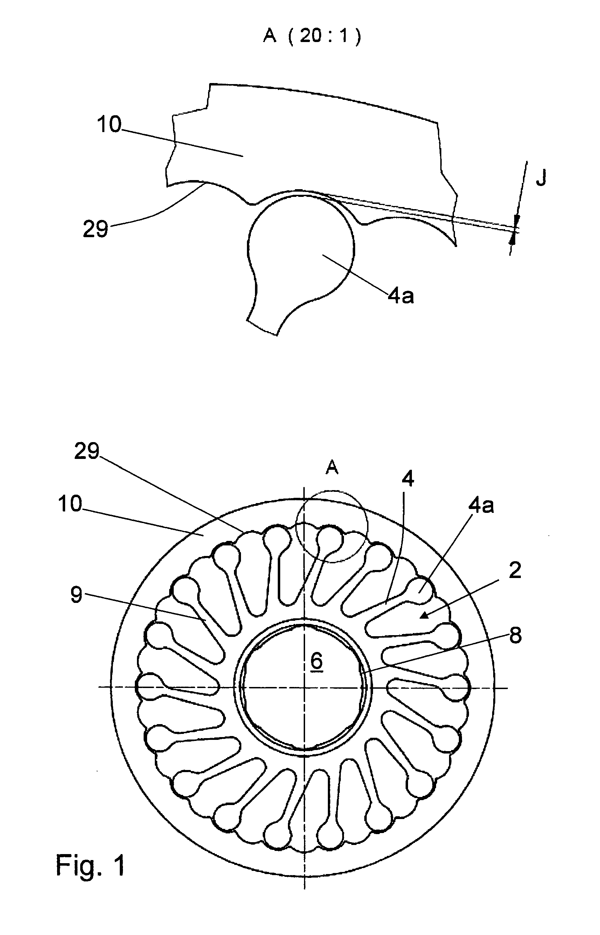

L'outil dynamométrique selon l'invention met en oeuvre un organe de limitation de couple qui est un organe élastique 2 représenté sur la figure 1. L'organe élastique 2 est de forme générale cylindrique et comporte, en son centre, une ouverture 6 structurée de manière à présenter une section non circulaire, non déformable élastiquement, et formant un organe femelle.

Plus particulièrement, l'organe élastique 2 comporte une pluralité de lamelles 4 dont la forme, compte tenu du matériau utilisé, est déterminée de manière à ce qu'elles soient déformables élastiquement selon une direction essentiellement radiale. Ces lamelles 4 sont orientées vers l'extérieur, principalement selon des directions non radiales et se terminent, dans un mode de réalisation préféré, par un patin comprenant, par exemple, une portion cylindrique 4a sensiblement orthogonale au plan général de l'organe élastique. Comme on le comprendra ci-après, c'est la déformation élastique des lamelles qui permet d'obtenir la limitation du couple. Contrairement à un organe de limitation de couple de l'état de la technique qui se déforme plastiquement, l'organe élastique 2 utilisé dans l'invention ne subit aucune transformation irréversible lorsque le couple maximal est atteint.

Comme on peut le voir sur la figure 1, l'ouverture 6 peut également être ménagée dans un moyeu 8 réalisé au moyen d'une deuxième pièce, solidaire d'une bague 9 portant les lamelles 4. Le moyeu est avantageusement réalisé en un matériau non déformable élastiquement, par exemple de type métallique, tel qu'un acier inox.

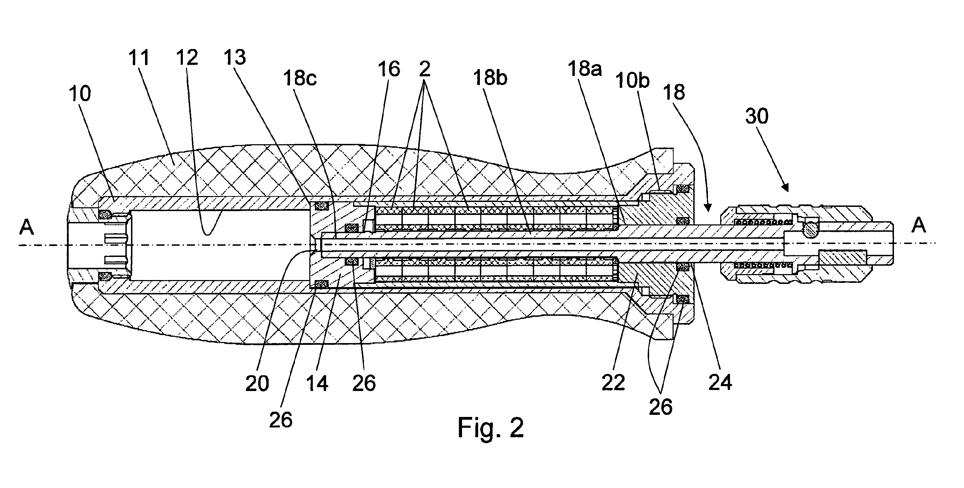

La figure 2 montre un corps de préhension 10, d'axe longitudinal AA, surmonté d'une poignée 11. Selon cet axe, le corps 10 est traversé par un canal cylindrique 12. Ce dernier comporte une butée 13 destinée à fournir un appui à un premier palier 14, présentant, en son centre, un logement 16 pour recevoir un arbre 18, qui sera décrit en détail ci-après. L'arbre 18 peut comporter, à son extrémité, un taraudage 20 d'axe AA destiné à coopérer avec une vis pour le rendre solidaire du palier 14. On peut, par exemple, accéder à la vis depuis l'extrémité du corps de préhension.

La deuxième extrémité du corps de préhension 10 est dotée d'un pas de vis 10b pour coopérer avec un deuxième palier 22, muni en son centre d'une ouverture 24 dans laquelle l'arbre est ajusté.

Au niveau de chacun des paliers 14 et 22 des joints 26 peuvent être insérés entre le palier et le corps de préhension 10, d'une part, et entre le palier et l'arbre 18, d'autre part.

On a également représenté sur la figure 2, un porte-instrument 30 de type connu de l'homme du métier, susceptible d'être rendu solidaire en rotation d'un instrument conformé pour coopérer avec un objet à visser. La partie du porte-instrument assurant la liaison avec l'instrument ne fait pas en soi partie de l'invention et ne sera pas décrite en détail.

Le porte-instrument 30 se prolonge par l'arbre 18 mentionné précédemment. Celui-ci est dimensionné de manière à pouvoir traverser l'ouverture 24, prendre place dans le canal 12 du corps de préhension 10, tandis que son extrémité libre prend place dans le logement 16. Plus précisément, l'arbre 18 comporte une première portion 18a ajustée à la dimension de l'ouverture 24. Puis, en s'éloignant de la partie du porte-instrument destinée à recevoir un instrument, une deuxième portion 18b destinée à être logée dans le canal 12. La deuxième portion 18b est structurée de manière à présenter une section non circulaire, et forme ainsi un organe mâle pouvant être lié en rotation avec l'organe femelle de l'organe élastique 2, qui est de forme correspondante. Enfin, l'arbre se termine par une troisième portion 18c ajustée aux dimensions du logement 16.

Le porte-instrument 30 est destiné à être monté pivotant, à friction, dans le canal 12, au moyen d'une pluralité d'organes élastiques 2 montés sur l'arbre 18 entre les deux paliers 14 et 22, interposé entre l'arbre 18 et la paroi du canal 12.

Pour assurer la friction entre le corps de préhension 10 et les organes élastiques 2, la paroi intérieure du canal 12 présente une succession de creux 29, orientés selon l'axe AA et dont la section, visible sur la figure 1 et particulièrement sur la vue rapprochée, est ovoïde. Les creux 29 occupent toute la longueur du canal 12 comprise entre les deux paliers 14 et 22, et sont destinés à recevoir les patins.

Les portions cylindriques 4a sont ainsi définies de manière à coopérer avec les creux 29 du canal 12. D'autres formes peuvent être choisies tant que les extrémités des lamelles 4 sont capables de coopérer avec les structures de la paroi du canal 12 pour créer une friction susceptible de contraindre les lamelles élastiques 4. Plus particulièrement, c'est la contrainte qu'il est nécessaire d'appliquer sur les lamelles élastiques 4 pour qu'elles passent d'un creux à un autre qui détermine le couple de serrage maximal.

Comme on peut le voir sur la figure 1, les creux 29 se succèdent de manière ininterrompue, de sorte que, entre deux creux 29 consécutifs, il n'existe pas de surface permettant à un patin d'occuper une position stable. Autrement dit, la paroi intérieure du canal 12 est conformée de sorte que seuls les creux 29 puissent recevoir les patins de manière stable. En effet, si cette caractéristique n'est pas respectée, il a été remarqué qu'il était possible que les organes élastiques 2 soient positionnés de manière stable avec les patins en appuis entre deux creux 29. Naturellement, dans une telle position, les lamelles élastiques 4 sont contraintes. Si une stérilisation est effectuée alors que les organes élastiques se trouvent ainsi contraints, leur dilatation causée par le chauffage de la stérilisation entraîne une modification des propriétés élastiques des organes 2 et un dérèglement du couple de serrage maximal.

Il a été remarqué que des outils présentant les caractéristiques ci-dessus pouvaient subir des fluctuations de leur couple maximal de serrage après qu'ils aient subi des traitements de stérilisation. Pour pallier cet inconvénient et selon un point important de l'invention, les organes élastiques 2 sont montés sur l'arbre 18, solidaires en rotation mais avec jeu. Tout d'abord, on veillera à ce qu'un jeu subsiste entre l'organe mâle formé par l'arbre 18 et l'organe femelle formé par l'ouverture 6. Ce jeu est typiquement compris entre 1 et 5 centièmes, de préférence de 3 centièmes. Ensuite, un jeu compris entre 2 et 15 centièmes, de préférence compris entre 5 et 10 centièmes est prévu entre les patins et les creux 29, lorsque les organes élastiques sont au repos. On notera que les organes élastiques 2 ne seraient pas fous sur l'arbre s'ils ne présentaient qu'un jeu entre les organes mâle et femelle. En effet, dans un tel cas, les lamelles 4 presseraient sur la paroi intérieure du canal 12, ce qui maintiendrait les organes élastiques. La conjugaison de ces jeux permet d'éviter tout phénomène d'hyperstaticité au niveau des organes élastiques 2. Ainsi, lorsque, lors des étapes de stérilisation, les organes élastiques 2 se dilatent sous l'effet de la chaleur, cette déformation se fait librement, sans création de contrainte excessive sur les organes élastiques qui, après refroidissement, conservent toutes leurs propriétés élastiques et nominales. Le couple de serrage maximum est ainsi parfaitement conservé.

Pour obtenir un alignement parfait des organes élastiques 2 en référence à l'axe AA et au corps de préhension 10, malgré les jeux mentionnés ci-dessus, le rôle et la précision des paliers 14 et 22 sont importants. En effet, seuls les paliers servent d'élément de guidage pour l'arbre 18. De plus, par son vissage, le deuxième palier 22 positionne les organes élastiques 2 selon l'axe AA.

Les organes élastiques 2 sont avantageusement réalisés en une matière ne nécessitant pas de lubrification et résistant aux traitements habituels de stérilisation, thermiques et par rayonnement. Différents tests ont permis de démontrer que les polymères du type polyéther-éther-cétone (connu sous le nom de PEEK) présentaient les caractéristiques requises. On pourra choisir, plus précisément du PEEK 151 G. On pourrait également réaliser le corps de préhension 10 en PEEK, ou en revêtir la paroi intérieure du canal 12, les organes élastiques étant réalisés dans une autre matière susceptible de présenter des propriétés élastiques convenables, en acier inox par exemple. L'homme du métier pourrait également envisager de réaliser les organes élastiques d'une part, et le corps de préhension d'autre part, en un seul matériau ne nécessitant pas de lubrification. Une telle possibilité est particulièrement avantageuse dans la mesure où elle supprime toute difficulté liée à des différences de coefficient de dilatation entre les organes élastiques 2 et le corps de préhension 10.

Typiquement, des organes élastiques tels que décrits ci-dessus et réalisés en PEEK permettent d'obtenir des valeurs de couple de serrage de l'ordre de quelques N.m, typiquement comprises entre 0.5 et 15 N.m.

Ainsi est obtenu un outil dont le réglage du couple maximal de serrage peut être particulièrement précis, avec une durée de vie améliorée. Non seulement, l'outil présente les avantages de certains outils de l'état de la technique, à savoir que la précision atteinte est de l'ordre de 3% pour 10 000 déclenchements. On définit un déclenchement comme étant le moment où le corps de préhension se désolidarise du porte-instrument, passant d'une première à une deuxième position relative de l'un de ces éléments par rapport à l'autre. L'outil n'a pas besoin d'être lubrifié et peut être étanche, évitant tout risque de contamination du patient. De plus, l'outil selon l'invention présente l'avantage essentiel de particulièrement bien se comporter lors des chauffages liés à des opérations de stérilisation. Même après des cycles de chauffage répétés, le couple maximal calibré en usine reste inchangé, ce qui garantit une utilisation extrêmement sûre et sécurisée.

## Revendications

1. Outil dynamométrique à usage médical d'axe longitudinal AA comportant:
- un corps de préhension (10) creux comprenant une paroi intérieure présentant une succession de creux (29) orientés selon l'axe AA et dont la section est ovoïde,
- un porte-instrument (30) pour être rendu solidaire en rotation d'un instrument conformé pour coopérer avec un objet à visser, ledit porte-instrument se prolongeant par un arbre (18) d'axe AA pivotant à l'intérieur du corps de préhension,
ledit porte-instrument (30) étant relié à friction audit corps de préhension au moyen d'une pluralité d'organes élastiques (2) disposés entre ledit arbre (18) et ledit corps de préhension (10), ladite friction permettant une limitation du couple entre le corps de préhension (10) et le porte-instrument (30),
chaque organe élastique (2) comportant une pluralité de lamelles (4) disposées principalement selon des directions non radiales et déformables élastiquement selon une direction essentiellement radiale, les extrémités des lamelles formant des patins destinés à coopérer avec lesdits creux (29) de ladite paroi intérieure, la coopération entre les lamelles (4) et lesdits creux (29) engendrant ladite friction lorsqu'un couple est appliqué entre ledit corps de préhension (10) et ledit porte-instrument (30),
**caractérisé en ce que** lesdits organes élastiques (2) sont montés fous sur l'arbre (4) et présentent, lorsqu'aucun couple n'est appliqué entre ledit corps de préhension et ledit porte-instrument, un jeu entre l'arbre (18) et lesdits organes élastiques (2), d'une part, et entre lesdits organes élastiques (2) et ladite paroi intérieure, d'autre part.

2. Outil selon la revendication 1, **caractérisé en ce que** lesdits patins sont de forme cylindrique et sont destinées à être sensiblement parallèle audit arbre (18).

3. Outil selon l'une des revendications 1 et 2, **caractérisé en ce que** le jeu entre lesdits patins et lesdits creux (29) est compris entre 2 et 15 centièmes, de préférence entre 5 et 10 centièmes, lorsque l'outil est au repos, et **en ce que** le jeu entre ledit arbre et lesdits organes élastiques est typiquement compris entre 1 et 5 centièmes, de préférence, de 3 centièmes, lorsque l'outil est au repos.

4. Outil selon l'une des revendications précédentes, **caractérisé en ce que** ledit arbre (18) est monté pivotant dans le corps de préhension (10) au moyen d'au moins un premier (14) et un deuxième (22) paliers, ajustés dans le corps de préhension, l'arbre étant ajusté dans lesdits paliers, lesdits organes élastiques (2) étant montés sur l'arbre (18), entre lesdits paliers (14, 22).

5. Outil selon la revendication 5, **caractérisé en ce que** le premier palier (14) est monté en buté à l'intérieur dudit canal (12), le deuxième palier (22) étant vissé sur le corps de préhension (10).

6. Outil selon l'une des revendications précédentes, **caractérisé en ce que** les creux (29) se succèdent de manière ininterrompue, de sorte que, entre deux creux (29) consécutifs, il n'existe pas de surface permettant à un patin d'occuper une position stable.

7. Outil selon l'une des revendications précédentes, **caractérisé en ce que** lesdits organes élastiques comportent un moyeu (8) réalisé en un matériau non déformable élastiquement et une bague (9) portant les lamelles (4) et montée solidairement autour du moyeu.

8. Outil selon l'une des revendications précédentes, **caractérisé en ce que** les lamelles élastiques (4) sont réalisées en PEEK.

9. Outil selon l'une des revendications précédentes, **caractérisé en ce que** la paroi intérieure dudit canal (12) est en PEEK.

10. Outil selon l'une des revendications précédentes, **caractérisé en ce que** le corps de préhension (10) est en PEEK.

## Claims

1. Dynamometric tool for medical use, of longitudinal axis AA, comprising
- a hollow gripping body (10) comprising an inner wall having a series of recesses (29) which are oriented along the axis AA and the cross-section of which is ovoid,
- an instrument holder (30) for being secured in rotation to an instrument which is shaped so as to cooperate with an object to be screwed, said instrument holder being extended by a shaft (18) of axis AA which pivots inside the gripping body,
said instrument holder (30) being frictionally connected to said gripping body by means of a plurality of elastic members (2) arranged between said shaft (18) and said gripping body (10), said friction making it possible to limit the torque between the gripping body (10) and the instrument holder (30),
each elastic member (2) comprising a plurality of blades (4) arranged principally in non-radial directions and elastically deformable in an essentially radial direction, the ends of the blades forming skids which are designed to cooperate with said recesses (29) of said inner wall, the cooperation between the blades (4) and said recesses (29) bringing about said friction when a torque is applied between said gripping body (10) and said instrument holder (30),
**characterised in that** said elastic members (2) are mounted idle on the shaft (4) and exhibit, when no torque is applied between said gripping body and said instrument holder, a clearance between the shaft (18) and said elastic members (2), on the one hand, and between said elastic members (2) and said inner wall, on the other hand.

2. Tool according to claim 1, **characterised in that** said skids are of cylindrical shape and are designed to be substantially parallel to said shaft (18).

3. Tool according to one of claims 1 and 2, **characterised in that** the clearance between said skids and said recesses (29) is between 2 and 15 hundredths, preferably between 5 and 10 hundredths, when the tool is at rest, and **in that** the clearance between said shaft and said elastic members is typically between 1 and 5 hundredths, preferably 3 hundredths, when the tool is at rest.

4. Tool according to one of the preceding claims, **characterised in that** said shaft (18) is mounted in a pivoting manner in the gripping body (10) by means of at least a first bearing (14) and a second bearing (22) which are adjusted in the gripping body, the shaft being adjusted in said bearings, said elastic members (2) being mounted on the shaft (18) between said bearings (14, 22).

5. Tool according to claim 5, **characterised in that** the first bearing (14) is mounted in abutment inside said channel (12), the second bearing (22) being screwed onto the gripping body (10).

6. Tool according to one of the preceding claims, **characterised in that** the recesses (29) succeed one another in an uninterrupted manner so that, between two consecutive recesses (29), there is no surface which enables a skid to occupy a stable position.

7. Tool according to one of the preceding claims, **characterised in that** said elastic members comprise a hub (8) made from an elastically non-deformable material and a ring (9) carrying the blades (4) and mounted integrally around the hub.

8. Tool according to one of the preceding claims, **characterised in that** the elastic blades (4) are made from PEEK.

9. Tool according to one of the preceding claims, **characterised in that** the inner wall of said channel (12) is made from PEEK.

10. Tool according to one of the preceding claims, **characterised in that** the gripping body (10) is made from PEEK.

## Patentansprüche

1. Dynamometrisches Werkzeug zur medizinischen Verwendung mit einer Längsachse AA, umfassend:
- einen hohlen Greifkörper (10), umfassend eine Innenwand, die eine Folge von Vertiefungen (29) aufweist, die gemäß der Achse AA ausgerichtet sind, und deren Querschnitt eiförmig ist,
- einen Instrumentenhalter (30), um in Drehung mit einem entsprechenden Instrument fest verbunden zu werden, um mit einem zu schraubenden Gegenstand zusammenzuarbeiten, wobei der Instrumentenhalter durch eine Welle (18) der Achse AA verlängert ist, die sich im Inneren des Greifkörpers dreht,
wobei der Instrumentenhalter (30) durch Reibung mit mithilfe einer Vielzahl von elastischen Organen (2) dem Greifköper verbinden ist, die zwischen der Welle (18) und dem Greifköper (10) angebracht sind, wobei die Reibung eine Begrenzung des Drehmoments zwischen dem Greifkörpers (10) und dem Instrumentenhalter (30) ermöglicht,
wobei jedes elastische Organ (2) eine Vielzahl von Lamellen (4) umfasst, die hauptsächlich gemäß nicht radialen Richtungen angebracht sind und gemäß einer Richtung elastisch verformbar sind, die im Wesentlichen radial ist, wobei die Enden der Lamellen Führungskugeln bilden, die dazu vorgesehen sind, mit den Vertiefungen (29) der Innenwand zusammenzuarbeiten, wobei die Zusammenarbeit zwischen den Lamellen (4) und den Vertiefungen (29) die Reibung erzeugt, wenn ein Drehmoment zwischen dem Greifkörper (10) und dem Instrumentenhalter (30) angewendet wird,
**dadurch gekennzeichnet, dass** die elastischen Organe (2) lose auf der Welle (4) angebracht sind und, wenn ein Drehmoment zwischen dem Greifkörper und dem Instrumentenhalter angewendet ist, ein Spiel zwischen der Welle (18) und den elastischen Organen (2) einerseits und zwischen den elastischen Organen (2) und der Innenwand, andererseits, aufweisen.

2. Werkzeug nach Anspruch 1, **dadurch gekennzeichnet, dass** die Führungskugeln eine zylindrische Form aufweisen und dazu bestimmt sind, im Wesentlichen parallel zur Welle (18) zu sein.

3. Werkzeug nach einem der Ansprüche 1 und 2, **dadurch gekennzeichnet, dass** das Spiel zwischen den Führungskugeln und den Vertiefungen (29) zwischen 2 und 15 Zentimetern liegt, vorzugsweise zwischen 5 und 10 Hundertsteln, wenn das Werkzeug in Ruhestellung ist, und **dadurch**, dass das Spiel zwischen der Welle und den elastischen Organen typischerweise zwischen 1 und 5 Hundertsteln liegt, vorzugsweise 3 Zentimetern, wenn das Werkzeug in Ruhestellung ist.

4. Werkzeug nach einem der vorhergehenden Ansprüche, **dadurch gekennzeichnet, dass** die Welle (18) beweglich im Greifkörper (10) mithilfe mindestens eines ersten (14) und eines zweiten (22) Lagers, angepasst im Greifkörper, angebracht ist, wobei die Welle in den Lagern angepasst ist, wobei die elastischen Organe (2) auf die Welle (18), zwischen den Lagern (14, 22) angebracht sind.

5. Werkzeug nach Anspruch 5, **dadurch gekennzeichnet, dass** das erste Lager (14) im Anschlag auf das Innere des Kanals (12)angebracht ist, wobei des zweite Lager (22) auf den Greifkörper (10) geschraubt ist.

6. Werkzeug nach einem der vorhergehenden Ansprüche, **dadurch gekennzeichnet, dass** die Vertiefungen (29) auf ununterbrochene Weise aufeinander folgen, so dass zwischen zwei aufeinander folgenden Vertiefungen (29) keine Fläche vorhanden ist, die es einer Führungskugel ermöglicht, eine stabile Position einzunehmen.

7. Werkzeug nach einem der vorhergehenden Anspruche, **dadurch gekennzeichnet, dass** die elastischen Organe eine Nabe (8) umfassen, hergestellt aus einem elastisch nicht verformbaren Material, und einem Ring (9), der die Lamellen (4) trägt und einstückig um die Nabe montiert ist.

8. Werkzeug nach einem der vorhergehenden Ansprüche, **dadurch gekennzeichnet, dass** die elastischen Lamellen (4) aus PEEK hergestellt sind.

9. Werkzeug nach einem der vorhergehenden Ansprüche, **dadurch gekennzeichnet, dass** die Innenwand des Kanals (12) aus PEEK ist.

10. Werkzeug nach einem der vorhergehenden Ansprüche, **dadurch gekennzeichnet, dass** der Greifkörper (10) aus PEEK ist.
